(19) Europäisches Patentamt
European Patent Office
Office européen des brevets

(11) EP 2 204 398 A1

(12) EUROPEAN PATENT APPLICATION
published in accordance with Art. 153(4) EPC

(43) Date of publication:
**07.07.2010 Bulletin 2010/27**

(21) Application number: **09750690.1**

(22) Date of filing: **20.05.2009**

(51) Int Cl.:
***C08G 69/48*** (2006.01) ***A61K 31/4745*** (2006.01)
***A61K 31/785*** (2006.01) ***A61K 47/48*** (2006.01)
***A61P 35/00*** (2006.01)

(86) International application number:
**PCT/JP2009/059637**

(87) International publication number:
**WO 2009/142328 (26.11.2009 Gazette 2009/48)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **23.05.2008 JP 2008135926**

(71) Applicant: **NanoCarrier Co., Ltd.**
**Kashiwa-shi, Chiba 277-0882 (JP)**

(72) Inventors:
- **HARADA, Mitsunori**
**Kashiwa-shi**
**Chiba 277-0882 (JP)**
- **SAITO, Hiroyuki**
**Kashiwa-shi**
**Chiba 277-0882 (JP)**
- **KATO, Yasuki**
**Kashiwa-shi**
**Chiba 277-0882 (JP)**

(74) Representative: **Swan, Elizabeth Mary et al**
**Withers & Rogers LLP**
**Goldings House**
**2 Hays Lane**
**London SE1 2HW (GB)**

# (54) CAMPTOTHECIN POLYMER DERIVATIVE AND USES OF THE SAME

(57) A polymer derivative of camptothecin is provided by a linkage between a hydroxyl group of camptothecin and a carboxyl group of a block copolymer, wherein the block copolymer includes polyethylene glycol and polyaspartic acid, the carboxyl group is in a side chain of the polyaspartic acid, and the linking is an ester linkage.

Fig.2

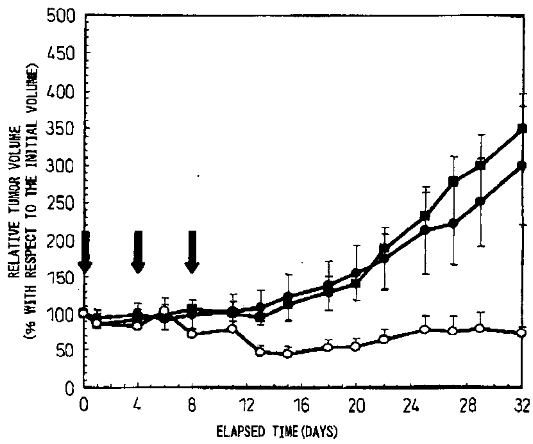


EP 2 204 398 A1

## Description

### TECHNICAL FIELD

The present invention relates to a polymer derivative of camptothecin and uses thereof.

### BACKGROUND ART

Camptothecin, an alkaloid extracted and isolated from *Camptotheca acuminata,* native to China, is an antitumor activity substance, which exhibits high antitumor activity and a broad antitumor spectrum. To test its use as a chemotherapeutic agent, a clinical trial was held in the early 1970s in the United States, but was terminated due to serious side effects. However, at the time of the clinical trial, the action mechanism of camptothecin was not clear since topoisomerase I had not been discovered, and since camptothecin is poorly-soluble in water, sodium camptothecin, which is in an open-circular form, was used. Research has revealed that the sodium camptothecin in an open-circular form has only a weak inhibition activity against topoisomerase I, suggesting that the then clinical trial was probably inappropriate (Non-patent Document 1). However, the results of the clinical trial have paved the way for development of various camptothecin derivatives, among which irinotecan hydrochloride and nogitecan hydrochloride are already in clinical use.

Recently, methods for solubilizing drugs having low water solubility have been examined. For example, a method has been developed in which a block copolymer having a hydrophilic segment and a hydrophobic segment are used to enclose a taxane anticancer drug by means of a hydrophobic interaction in a polymer micelle, thereby improving water solubility (Patent Documents 1 and 2). Patent Document 3 discloses that amido-linking doxorubicin hydrochloride to a block copolymer including polyethylene glycol and polyaspartic acid may enhance the solubility of drugs. Patent Document 4 discloses a polymer derivative including a block copolymer containing polyethylene glycol and polyglutamate to which a phenolic hydroxyl group of SN-38 is ester-linked. Patent Document 5 discloses camptothecin derivatives wherein camptothecins are linked to polyglutamate.

### PRIOR ART REFERENCES

#### Patent Documents

Patent Document 1: EP1127570 A
Patent Document 2: WO2004/082718
Patent Document 3: JP02-300133 A
Patent Document 4: WO2004/039869
Patent Document 5: WO01/70275

#### Non-patent Documents

Non-patent Document 1: Slichenmyer et al., The Current Status of Camptothecin Analogues as Antitumor Agent., Journal of the National Cancer Institute, Vol. 85, No. 4 (1993)

### DISCLOSURE OF THE INVENTION

### PROBLEMS TO BE SOLVED BY THE INVENTION

The present invention is intended to reduce the side effects of camptothecin and improve the pharmacological effects of camptothecin.

### MEANS FOR SOLVING THE PROBLEMS

The present inventors have developed a more useful anticancer agent, i.e., a novel polymer derivative of camptothecin provided by linking a hydroxyl group of camptothecin to a carboxyl group of a block copolymer, wherein the block copolymer includes polyethylene glycol and polyaspartic acid, the carboxyl group is in a side chain of the polyaspartic acid, and the linking is an ester linkage. The present inventors have further found that the polymer derivative of camptothecin has reduced side effects and improved effects, thereby having completed the present invention.

Accordingly, the present invention relates to the following aspects:

(1) A polymer derivative of camptothecin provided by a linkage between a hydroxyl group of camptothecin and a carboxyl group of a block copolymer, wherein the block copolymer includes polyethylene glycol and polyaspartic acid, the carboxyl group is in a side chain of the polyaspartic acid, and the linking is an ester linkage.

(2) The polymer derivative of camptothecin according to (1), represented by Formula (I):

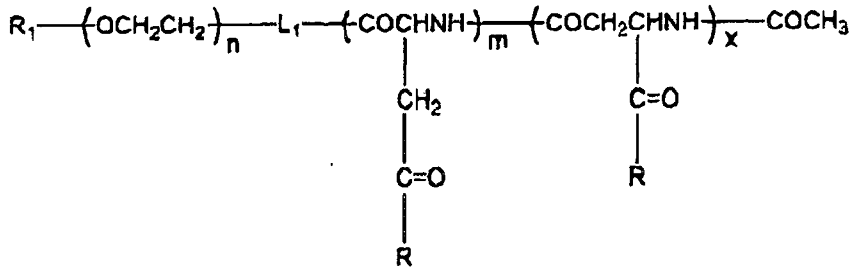

(I)

wherein

$R_1$ is a hydrogen atom or $C_1$-$C_6$ alkyl group;

$L_1$ is a linking group;

R is a hydroxyl group or the following structure:

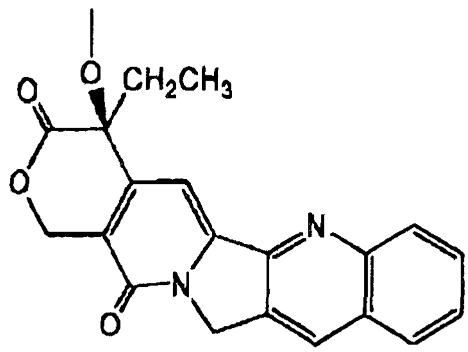

;

n is an integer of 40 to 450; and

m+x is an integer of 20 to 80;

0% to 90% of m+x is x;

wherein when x exists, the "COCHNH" units and the "$COCH_2CHNH$" units are disposed randomly.

(3) An anticancer agent including the polymer derivative of camptothecin according to (1) or (2).

BRIEF DESCRIPTION OF THE DRAWINGS

Fig. 1 is a graph showing changes in the plasma concentration of the drug after administration of a CPT polymeric derivative (PEG-pAsp-CPT) or a CPT solution. A filled circle represents the total concentration of CPT in a rat to which PEG-pAsp-CPT was intravenously administered; an open circle represents the concentration of CPT released from PEG-pAsp-CPT; and a filled triangle represents the plasma concentration of the drug in a rat to which the CPT solution was administered.

Fig. 2 is a graph showing the anticancer activity of a CPT polymeric derivative (PEG-pAsp-CPT) against a human prostate cancer cell PC-3 tumor-bearing animal. Each arrow represents the timing of administration; the ordinate indicates the relative tumor volume with respect to that observed when the administration was started; and the abscissa indicates the period of time (days). A filled circle represents a control group (untreated); a filled square represents a group wherein the CPT solution was administered; and an open circle represents a group wherein a CPT polymeric derivative was administered.

Fig. 3 is a graph showing changes in body weight after administration of a CPT polymeric derivative (PEG-pAsp-CPT) to a human prostate cancer cell PC-3 tumor-bearing animal. Each arrow represents the timing of administration;

the ordinate indicates the rate of change in body weight relative to that observed when the administration was started; and the abscissa indicates the period of time (days). A filled circle represents a control group (untreated); a filled square represents a group wherein the CPT solution was administered; and an open circle represents a group wherein a CPT polymeric derivative was administered.

Fig. 4 is a graph showing the anticancer activity of a CPT polymeric derivative (PEG-pAsp-CPT) against a human prostate cancer cell PC-3 tumor-bearing animal. Each arrow represents the timing of administration; the ordinate indicates the relative tumor volume with respect to that observed when the administration was started; and the abscissa indicates the period of time (days). A filled circle represents a control group (untreated); a filled square represents a group wherein 10 mg/kg of CPT polymeric derivative (in terms of CPT) was administered; and a filled triangle represents a group wherein 7 mg/kg of CPT polymeric derivative (in terms of CPT) was administered.

Fig. 5 is a graph showing changes in body weight after administration of a CPT polymeric derivative (PEG-pAsp-CPT) to a human prostate cancer cell PC-3 tumor-bearing animal. Each arrow represents the timing of administration; the ordinate indicates the rate of change in body weight relative to that observed when the administration was started; and the abscissa indicates the period of time (days). A filled circle represents a control group (untreated); a filled square represents a group wherein 10 mg/kg CPT of polymeric derivative (in terms of CPT) was administered; and a filled triangle represents a group wherein 7 mg/kg of CPT polymeric derivative (in terms of CPT) was administered.

## BEST MODE FOR CARRYING OUT THE INVENTION

The polymer derivative of camptothecin according to the present invention is **characterized in that** it is provided by a linkage between a hydroxyl group of camptothecin and a carboxyl group of a block copolymer, wherein the block copolymer comprises polyethylene glycol and polyaspartic acid, the carboxyl group is in a side chain of the polyaspartic acid, and the linking is an ester linkage.

In Formula (I) of the present invention, n represents an integer of 40 to 450, preferably an integer of 60 to 410, more preferably an integer of 110 to 340. m+x represents an integer of 20 to 80, especially preferably an integer of 25 to 50. Needless to say, the values for n, m and x are average values.

The $L_1$ linking group in the general formula of the present invention may be any group as long as it can link polyethylene glycol and polyaspartic acid, and preferably $R_5(CH_2)_pR_6$, wherein $R_5$ represents O, $R_6$ represents NH and p represents an integer of 1 to 6.

The polymer derivative of camptothecin according to the present invention may form a polymer micelle with an outer shell of polyethylene glycol in water. In this case, the particle size of the polymer micelle is preferably not more than 10 μm, and more preferably not more than 5 μm in view of administration to the human body. When the micelle is intended for intravenous administration, the particle size is preferably not more than 200 nm, and more preferably not more than 100 nm.

The block copolymer being a support to which camptothecin is linked may be synthesized by any method as long as it can produce a desired block copolymer. For example, the block copolymer can be obtained by the method described in Patent Document 3. According to this method, e.g., MeO-PEG-$CH_2CH_2CH_2$-$NH_2$ is used as an initiator, and N-carboxy-β-benzyl-L-aspartate (BLA-NCA) is fed to a dehydrated organic solvent and allowed to react until a desired degree of polymerization (the number of amino acid units, i.e., m + x in the formula) is reached, followed by removal of the benzyl group by alkaline hydrolysis.

Examples of a formulation including the polymer derivative of camptothecin according to the present invention include liquids and lyophilized formulations, the latter being preferred. In any case, the formulation may also include diluents, excipients, isotonic agents, and/or pH adjusters which are generally used in formulation.

The administration route of the polymer derivative of camptothecin according to the present invention is not limited, and preferably be parenteral administration such as subcutaneous, intravenous, intraarterial and topical administration, especially preferably intravenous administration.

The dose of the polymer derivative of camptothecin according to the present invention may be appropriately selected, depending on its usage; the age and sex of the patient; the disease severity of the patient; and other conditions. Although not being limited, the polymer derivative is usually administered at a dose of 0.1 to 1000 $mg/m^2$, preferably 1 to 500 $mg/m^2$, and more preferably 5 to 100 $mg/m^2$ per day in terms of camptothecin.

## EXAMPLES

The present invention will now be described concretely by way of Examples, but the scope of the present invention is not limited thereto. It should be noted that hereinafter, camptothecin will be referred to as CPT; and a polymer derivative of camptothecin will be referred to as PEG-pAsp-CPT.

Example 1

Synthesis of PEG-pAsp-CPT

In 80 mL of anhydrous DMF, 1 g of a methoxypolyethylene glycol-polyaspartic acid block copolymer (wherein one of the termini of polyaspartic acid is acetylated), PEG-pAsp-Ac (the average molecular weight of PEG: 12 kDa; the average number of aspartic acid residues: 40; carboxylic-type aspartic acid), was dissolved. To the resulting solution, 387 mg of camptothecin (Wako Pure Chemical Industries, Ltd.) was added. Subsequently, 823 mg of N,N'-dicyclohexylcarbodiimide (Kokusan Chemical Co., Ltd.) and 136 mg of dimethylaminopyridine (Wako Pure Chemical Industries, Ltd.) were added thereto in turn. The resulting mixture was stirred at 4°C for 3 days and allowed to warm to room temperature, followed by further stirring overnight. The reaction solution was transferred into a dialysis tube (Spectrum Laboratories, Spectra/Por (registered trademark), cut-off molecular weight: 3500) and dialyzed against 3 L of purified water at 4°C overnight. After removing precipitates, the obtained yellow solution was filtered through a 0.8 μm filter (Millipore, MILLEX(registered trademark)-AA), The filtrate was lyophilized to obtain 920 mg of a pale yellow powder. From the obtained powder, a 5 mg aliquot was weighed and dissolved into 1 mL of purified water. Based on the absorbance at 370 nm of the solution, the content of camptothecin was calculated to be 5.4% (the weight ratio of camptothecin to the polymer derivative of camptothecin).

Changes of Concentration in Blood Plasma

Example 2

Test for Blood Kinetics Using PEG-pAsp-CPT in Rat

1) Preparation of Polymeric Micelle

In a sample vial, 50 mg of the complex obtained in Example 1 which was weighed precisely was placed, and 1 mL of purified water was added thereto, followed by suspending the polymer. The resulting suspension was stirred for one day and night, and sonicated using Biodisruptor (Nihonseiki Kaisha Ltd., High Power Unit) with ice cooling for 10 minutes, followed by filtration through a 0.22 μm filter (Millipore, MILLEX (registered trademark) GP PES) and recovery of the resulting filtrate. The filtrate was subjected to gel filtration [GE Healthcare Bio-Sciences KK, PD-10, eluent: 20 mM sodium phosphate buffer (pH 7.4)/5% glucose], and the recovered micelle fraction (the average particle size: 80 nm) was used for the following experiment.

2) Animal Experiment

The above-described polymer micelle was administered to a male Wister rat (Charles River Laboratories Japan) through the tail vein under anesthesia with ether under the following conditions (n=1). Blood was collected with a heparin-coated syringe, and then blood plasma was recovered through a blood centrifugation at 4°C and stored at -30°C until measurement.

Dose: 1 mg/kg PEG-pAsp-CPT (in terms of CPT)
Timing of collection of blood: 5 minutes, 1 hour and 3 hours after the administration
Amount of blood collection : 0.2 to 0.25 mL/collection (from the jugular vein)

For comparison, a solution of camptothecin (Wako Pure Chemical Industries, Ltd.) in DMSO was administered followed by collection of blood, in the same manner as' described above.

3) Measurement of CPT Concentration in Blood Plasma

a) Measurement of Total CPT Concentration

To 50 μL of the collected rat blood plasma, 2 volumes (100 μL) of 6N HCl was added, and the resulting mixture was heated at 100°C for 1 hour in a Cryotube (Ieda Chemical Co., Ltd.). After recovery of 75 μL of the reaction solution, 50 μL of 6N NaOH was added thereto to neutralize the solution, and the resulting mixture was filtered through a 0.22 μm filter (Millipore, MILLEX (registered trademark) -Gv). The filtrate was 10-fold diluted with 25 mM ammonium formate buffer (pH 3.0), and the treated sample solution was filled in an HPLC sample vial, to determine the total CPT concentration

in the blood plasma by HPLC analysis.

b) Measurement of Free CPT Concentration

To 30 μL of the collected rat blood plasma, 100 μL of acetonitrile and 20 μL of 10 mM HCl were sequentially added, and the resulting mixture was stirred. Subsequently, the resulting solution was subjected to centrifugation (Funakoshi, Chibitan, 10,000 rpm, 10 minutes) at 4°C, and 50 μL of the supernatant was collected. To 50 μL of this supernatant, 100 μL of ammonium formate buffer (pH 3.0) was added, and the resulting mixture was filtered through a 0.22 μm filter (Millipore, MILLEX (registered trademark) -GV). The filtrate was filled in an HPLC sample vial, and the concentration of free CPT in the blood plasma was determined.

c) Condition of Analysis

The HPLC conditions were as followed. Unless otherwise specified, the HPLC analysis of CPT was carried out under the same conditions.

System: Waters Alliance System
Column: Tosoh TSK-gel ODS-80TM (Φ4.6 × 150 mm) (40°C)
Mobile phase: 25 mM ammonium formate buffer (pH 3.0)/acetonitrile=70/30
Flow rate: 1 mL/min.
Detection: fluorescence (Ex: 370 nm, Em: 420 nm) Injection volume: 20 μL

4) Results of Measurement of Changes in Plasma Concentration with Time

In the case of administration of the solution, the blood concentration decreased rapidly. In the case of PEG-pAsp-CPT, the total CPT concentration remained high in the blood plasma. Although being lower than the total CPT concentration, the free CPT concentration changed within the range of several to ten times higher than that in the case of administration of the solution. The results are shown in Figure 1.

Evaluation of Pharmacological Effect

Example 3

Test for Pharmacological Effect Using Human Prostate Cancer PC-3 Cells - 1

PC-3 cells (purchased from ATCC via Summit Pharmaceuticals International Corporation; which provision is also applied hereinafter) were cultured using RPMI1640 + 10% FBS medium under 5% $CO_2$ at 37°C to allow their growth to attain the cell number required for transplantation. The cells were suspended in physiological saline and subcutaneously inoculated to the back of male nude mice [Blab nu/nu, 8 weeks old, Charles River Laboratories Japan] such that the dose of $3 \times 10^6$ cells/100 μL per mouse was attained. Thereafter, the nude mice were kept for 7 days, and when the tumor volume reached 83.3 t 6.2 $mm^3$ (average ± SE, wherein SE indicates standard error; which provision is also applied hereinafter), administration of drugs were started. A total of 3 times of administration through the tail vein at intervals of 4 days were scheduled, and changes in the tumor volume and body weight with time were measured for 3 groups (n=7), that is, (1) control (untreated); (2) the CPT DMSO solution 7 mg/kg; and (3) PEG-pAsp-CPT 3 mg/kg (the dose is based on the amount of camptothecin (mg/kg/injection)). The major diameter (a mm) and the minor diameter (b mm) of each tumor were measured using an electronic caliper (Mitutoyo Corporation), and the tumor volume was calculated according to the following equation.

$$\text{Tumor volume } (mm^3) = a \times b^2 / 2$$

Changes in the tumor volume with time after starting of administration of the samples are shown in Fig. 2, while changes in body weight are shown in Fig. 3. In the case of administration of the CPT solution, tumor growth inhibition effect was hardly observed in comparison with the control group, but the body weight decreased by 10% or more at maximum. On the other hand, in the case of PEG-pAsp-CPT, in spite of its dose not more than a half of that of the CPT solution, growth of the tumor was inhibited, and decrease in body weight was not observed. From the above results, it was revealed that

PEG-pAsp-CPT has a superior therapeutic effect compared to the solution.

Example 4 .

Test for Pharmacological Effect Using Human Prostate Cancer PC-3 Cells - 2

PC-3-cells were cultured using RPMI1640 + 10% FBS medium under 5% $CO_2$ at 37°C to allow their growth to attain the cell number required for transplantation. The cells were suspended in physiological saline and subcutaneously inoculated to the back of male nude mice [Balb nu/nu, 5 weeks old, Charles River Laboratories Japan] such that the dose of $3 \times 10^6$ cells/100 μL per mouse was attained, Thereafter, the nude mice were kept for 15 days, and when the tumor volume reached 81.6 ± 5.0 $mm^3$ (average ± SE), administration of drugs were started. A total of 3 times of administration through the tail vein at intervals of 4 days were scheduled, and changes in the tumor volume and body weight with time were measured for the following 3 groups (n=8). (1) Control (untreated); (2) PEG-pAsp-CPT 7 mg/kg; and (3) PEG-pAsp-CPT 10 mg/kg (the doses in (1) and (2) are based on the amount of CPT (mg/kg/injection)).

Changes in the tumor volume with time after starting of administration of the samples are shown in Fig. 4, and changes in body weight are shown in Fig. 5. In the group wherein 7 mg/kg of PEG-pAsp-CPT was administered, growth of the tumor was effectively inhibited. In addition, decrease in body weight was up to about 10%, and 15 days after starting of the administration, body weight was recovered to the same level as that of the control group, and there were no cases of death. When the dose was 10 mg/kg, the tumor was further decreased in size, but body weight decreased, and 5 cases out of the 8 cases died. The above results have confirmed that the polymer derivative of CPT, PEG-pAsp-CPT, shows reduced side effects and has excellent therapeutic effects.

## Claims

1. A polymer derivative of camptothecin provided by a linkage between a hydroxyl group of camptothecin and a carboxyl group of a block copolymer, wherein the block copolymer comprises polyethylene glycol and polyaspartic acid, the carboxyl group is in a side chain of the polyaspartic acid, and the linking is an ester linkage.

2. The polymer derivative of camptothecin according to claim 1, represented by Formula (I):

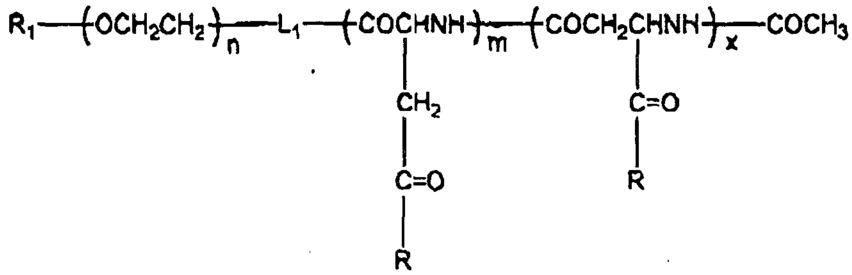

(I)

wherein
$R_1$ is a hydrogen atom or $C_1$-$C_6$ alkyl group;
$L_1$ is a linking group;
R is a hydroxyl group or the following structure:

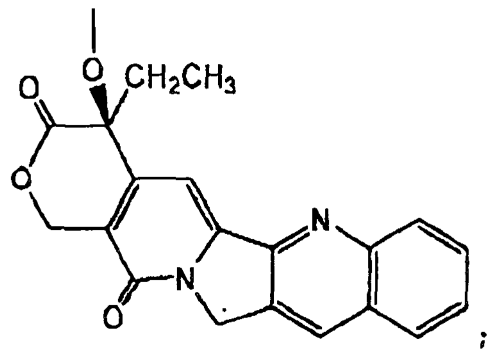

n is an integer of 40 to 450; and
m+x is an integer of 20 to 80;
0% to 90% of m+x is x;
wherein when x exists, the "COCHNH" units and the "$COCH_2CHNH$" units are arranged randomly.

**3.** An anticancer agent comprising the polymer derivative of camptothecin according to claim 1 or 2.

Fig.1

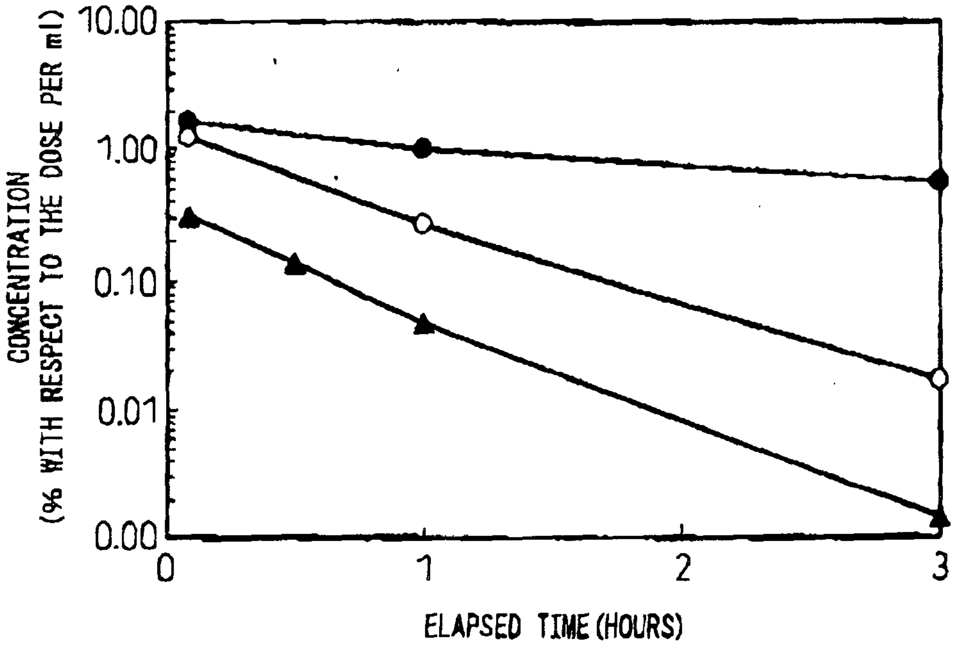
CONCENTRATION
(% WITH RESPECT TO THE DOSE PER ml)
10.00
1.00
0.10
0.01
0.00
0
1
2
3
ELAPSED TIME (HOURS)

Fig.2

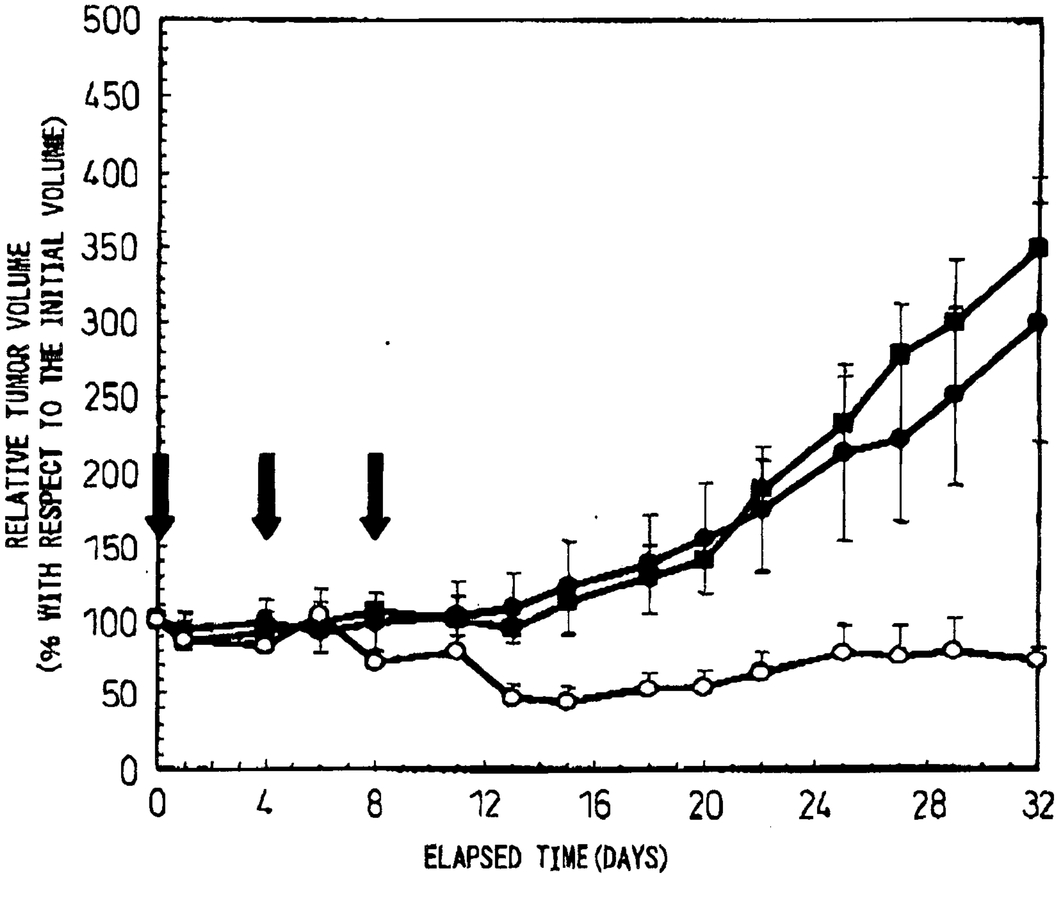
RELATIVE TUMOR VOLUME
(% WITH RESPECT TO THE INITIAL VOLUME)
500
450
400
350
300
250
200
150
100
50
0
0
4
8
12
16
20
24
28
32
ELAPSED TIME (DAYS)

Fig.3

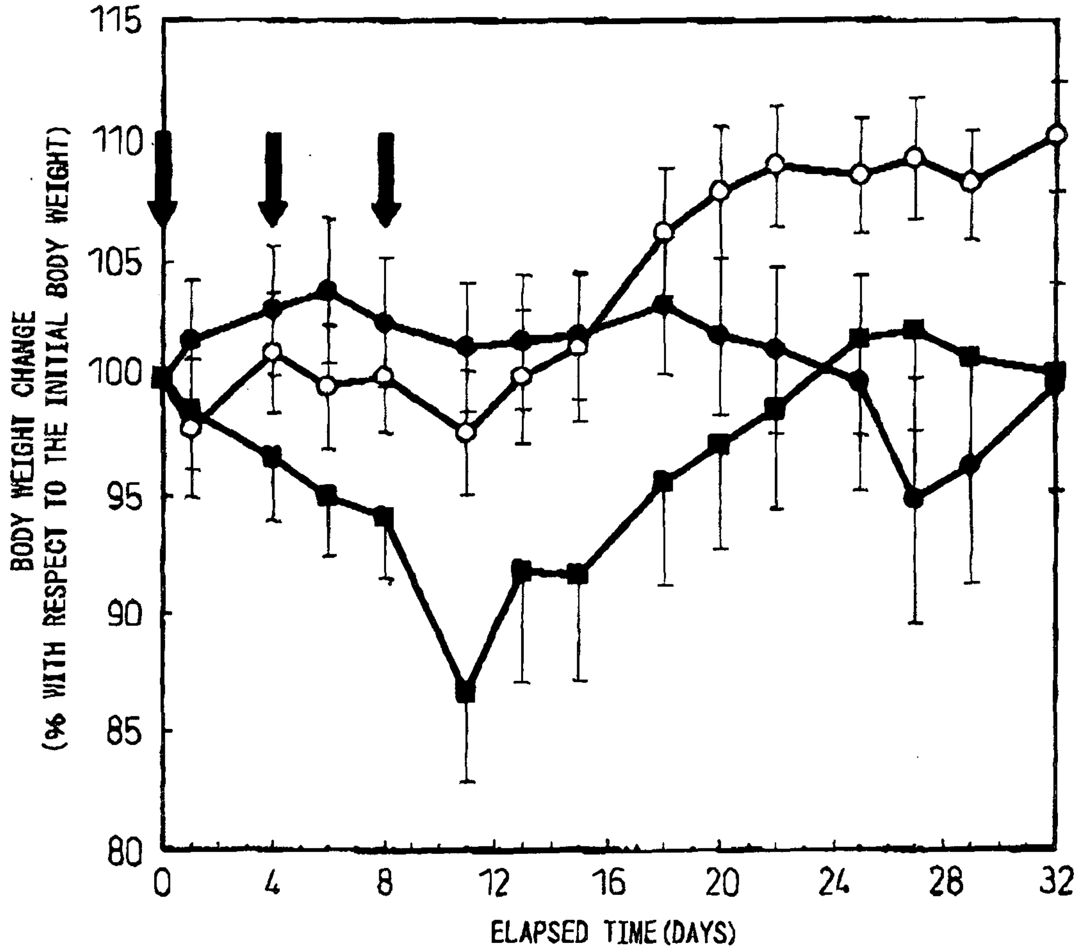
115
110
105
100
95
90
85
80
BODY WEIGHT CHANGE
(% WITH RESPECT TO THE INITIAL BODY WEIGHT)
0
4
8
12
16
20
24
28
32
ELAPSED TIME (DAYS)

Fig.4

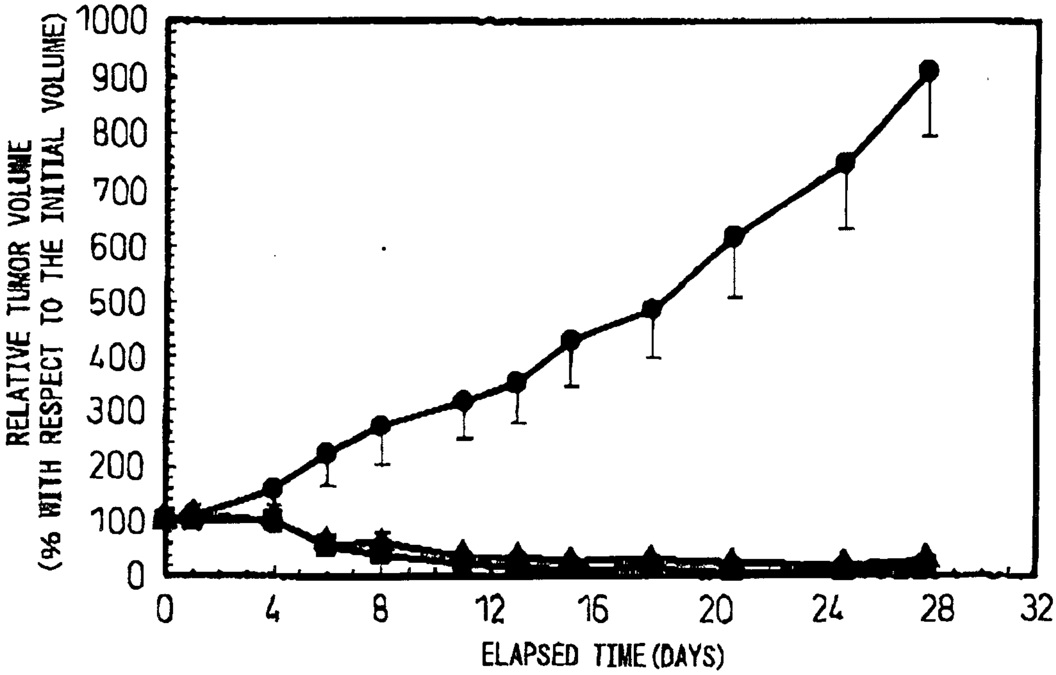
RELATIVE TUMOR VOLUME
(% WITH RESPECT TO THE INITIAL VOLUME)
1000
900
800
700
600
500
400
300
200
100
0
0
4
8
12
16
20
24
28
32
ELAPSED TIME (DAYS)

Fig.5

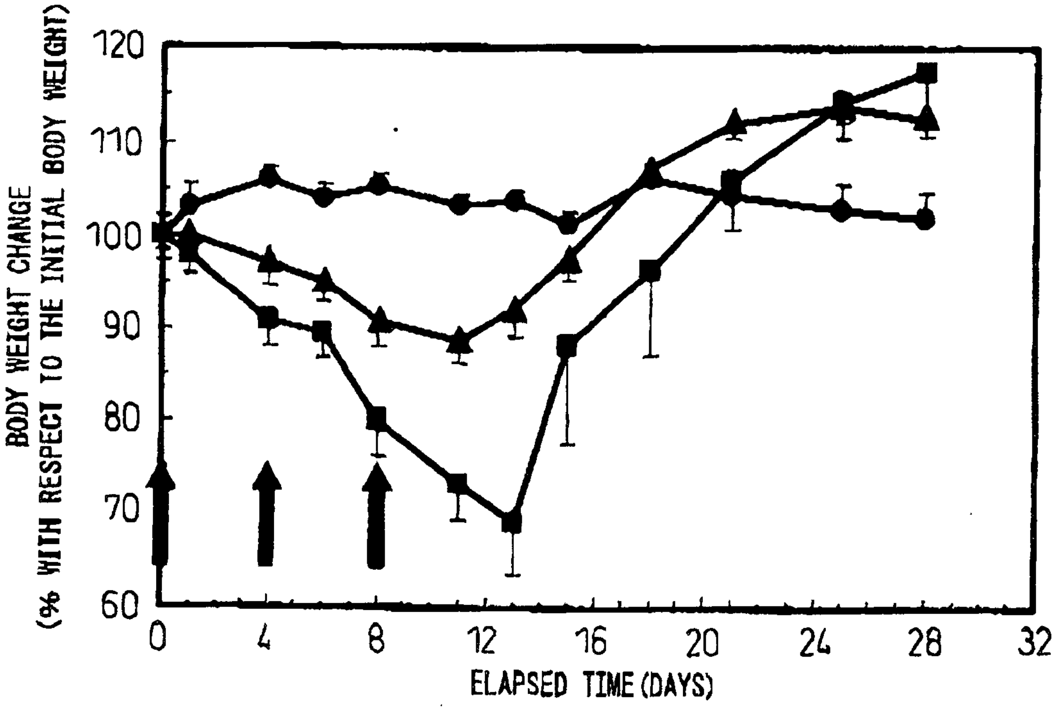
BODY WEIGHT CHANGE
(% WITH RESPECT TO THE INITIAL BODY WEIGHT)
120
110
100
90
80
70
60
0
4
8
12
16
20
24
28
32
ELAPSED TIME (DAYS)

## INTERNATIONAL SEARCH REPORT

International application No.
PCT/JP2009/059637

A. CLASSIFICATION OF SUBJECT MATTER
*C08G69/48*(2006.01)i, *A61K31/4745*(2006.01)i, *A61K31/785*(2006.01)i, *A61K47/48*(2006.01)i, *A61P35/00*(2006.01)i

According to International Patent Classification (IPC) or to both national classification and IPC

B. FIELDS SEARCHED

Minimum documentation searched (classification system followed by classification symbols)
C08G69/48, A61K31/4745, A61K31/785, A61K47/48, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2004/039869 A1 (Nippon Kayaku Co., Ltd.), 13 May, 2004 (13.05.04), Claims 1 to 6; page 22, lines 9 to 12<br>& US 2006/0067910 A1 & EP 1580216 A1<br>& CA 2502870 A & KR 10-2005-0070103 A<br>& CN 1708540 A & RU 2315782 C | 1,3<br>2,3 |
| Y | JP 2005-519122 A (Beijing Jiankai Technology Co., Ltd.), 30 June, 2005 (30.06.05), Claims 1 to 4, 10 to 14; Par. Nos. [0044], [0045]; example 5<br>& US 2005/0147617 A1 & EP 1489125 A1<br>& WO 2003/074586 A1 & CN 1442440 A | 2,3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>06 July, 2009 (06.07.09) | Date of mailing of the international search report<br>14 July, 2009 (14.07.09) |
| Name and mailing address of the ISA/<br>Japanese Patent Office<br>Facsimile No. | Authorized officer<br>Telephone No. |

Form PCT/ISA/210 (second sheet) (April 2007)

**INTERNATIONAL SEARCH REPORT**

International application No.
PCT/JP2009/059637

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-527443 A (Cell Therapeutics, Inc.), 16 September, 2003 (16.09.03), Claims 1, 9; example 1<br>& US 2002/0016285 A1 & EP 1267939 A<br>& WO 2001/070275 A2 & AU 4751301 A<br>& NO 20024421 A & HU 204562 A<br>& CA 2402643 A & SK 14822002 A<br>& BR 109272 A & PL 358335 A<br>& CZ 20023330 A & IL 151685 D<br>& CN 1429121 A | 2,3 |
| A | JP 2-300133 A (Research Development Corp. of Japan), 12 December, 1990 (12.12.90), Claims; page 4, upper left column, line 8 to upper right column, line 6<br>& US 5412072 A & EP 397307 A3<br>& DE 69029006 C & AU 5469990 A<br>& ES 2098247 T & KR 10-1992-0006912 B<br>& CA 2016505 A1 | 1-3 |
| A | JP 5-124969 A (Research Development Corp. of Japan), 21 May, 1993 (21.05.93), Claims 1 to 3; Par. Nos. [0012], [0024]; examples 1 to 3<br>(Family: none) | 1-3 |
| A | JP 5-117385 A (Research Development Corp. of Japan), 14 May, 1993 (14.05.93), Claims 1, 3, 9; Par. No. [0019]; examples 1 to 4<br>(Family: none) | 1-3 |
| A | JP 5-955 A (Nippon Kayaku Co., Ltd.), 08 January, 1993 (08.01.93), Claims 1 to 3; examples<br>(Family: none) | 1-3 |
| A | JP 2000-507930 A (PG-TXL Co., L.P.), 27 June, 2000 (27.06.00), Full text<br>& US 5977163 A & EP 932399 A<br>& WO 1997/033552 A1 & DE 69735057 D<br>& NO 984210 A & PL 328807 A<br>& AU 2580697 A & CA 2250295 A<br>& BR 9710646 A & HU 9903952 A<br>& NZ 332234 A & NO 20072562 A<br>& CZ 9802908 A & IL 126179 D<br>& AU 735900 B & EA 2400 B<br>& AT 314843 T | 1-3 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1127570 A
- WO 2004082718 A
- JP 2300133 A
- WO 2004039869 A
- WO 0170275 A

### Non-patent literature cited in the description

- **Slichenmyer et al.** The Current Status of Camptothecin Analogues as Antitumor Agent. *Journal of the National Cancer Institute,* 1993, vol. 85 (4